# EUROPEAN PATENT APPLICATION

(11) **EP 4 732 882 A1**
(43) Date of publication of application: **29.04.2026**
(21) Application number: 24825931.9
(22) Date of filing: 19.06.2024
(51) Int. Cl.: A61M 25/10, A61M 25/14

(54) **BALLOON CATHETER**

(30) Priority: 22.06.2023 JP 2023102824
(71) Applicant: KANEKA CORPORATION, Osaka 530-8288 (JP)
(72) Inventor: MATSUFUJI, Kazuki, Settsu-shi, Osaka 566-0072 (JP); KOJIMA, Masahiro, Settsu-shi, Osaka 566-0072 (JP); HAMABUCHI, Takahisa, Osaka-shi, Osaka 530-8288 (JP)
(74) Representative: Plasseraud IP
(86) International application number: PCT/JP2024/022201
(87) International publication number: WO 2024/262530

(57) **Abstract**

An object is to provide a balloon catheter having a first balloon, a second balloon, a first inflation tube having a lumen in communication with a lumen of the first balloon, and a second inflation tube having a lumen in communication with a lumen of the second balloon, the balloon catheter making it less likely for a kink to be generated even when being inserted into a bent portion of a blood vessel.

A balloon catheter including: a shaft, a first balloon, a second balloon, a first inflation tube, and a second inflation tube, in which the first inflation tube has a lumen in communication with the lumen of the shaft and a lumen of the first balloon, the second inflation tube has a lumen in communication with the lumen of the shaft and a lumen of the second balloon, the shaft has a first section which is present on a distal side and in which the first and the second inflation tubes are disposed and a second section which is present proximal to the first section and in which the first and the second inflation tubes are disposed, at least two of the shaft, the first inflation tube, and the second inflation tube are fixed to each other in the first section, and the shaft, the first inflation tube, and the second inflation tube are not fixed to each other in the second section.

## Description

### TECHNICAL FIELD

The present invention relates to a balloon catheter.

### BACKGROUND ART

Diseases such as angina pectoris and myocardial infarction are caused by the formation of stenosis sites hardened by calcification and other factors in the inner walls of blood vessels. One of the treatment methods for these diseases is angioplasty, in which a balloon catheter is used to dilate the stenosis site. Such angioplasty is sometimes also called Percutaneous Transluminal Angioplasty (PTA) or Percutaneous Transluminal Coronary Angioplasty (PTCA). Angioplasty is a minimally invasive therapy that does not require a thoracotomy such as a bypass surgery, and is widely conducted.

A balloon catheter to be used for angioplasty at least has: a shaft extending in a longitudinal direction from a proximal side to a distal side; a balloon disposed at a distal part of the shaft; and an inflation tube which is in communication with the balloon and through which a fluid for inflating or deflating the balloon can flow. The balloon is inserted into a blood vessel and delivered to a stenosis site through the inside of the blood vessel in a deflated state. After the balloon is delivered to the stenosis site, a fluid is introduced into or discharged from the inflation tube using an indeflator (balloon pressurizer) connected to the inflation tube, thereby controlling inflation and deflation of the balloon. An example of such a balloon catheter is described in Patent document 1. FIG. 42 of Patent document 1 shows an inflation device including an inner balloon member and a plurality of outer balloon members, and FIG. 43A shows a state where the outer balloon members are inflated.

### RELATED ART DOCUMENT

### PATENT DOCUMENT

Patent Document 1: U.S. Patent Application Publication No. 2012/0209375 Specification

### SUMMARY OF THE INVENTION

### PROBLEMS TO BE SOLVED BY THE INVENTION

The balloon catheter described in Patent document 1 includes an inner balloon member and a plurality of outer balloon members, and Patent document 1 describes that inflation lumens respectively corresponding to the inner balloon member and the plurality of outer balloon members are provided in order to inflate the inner balloon member and the outer balloon members at different timings. However, a region in which the plurality of inflation lumens are disposed has a rigidity higher than a rigidity of a region in which the plurality of inflation lumens are not disposed, and the difference between the rigidities becomes large in the longitudinal direction. Consequently, for example, insertion of the balloon catheter into a bent portion of a blood vessel leads to generation of a kink between the section in which the plurality of inflation lumens are disposed and the section in which the plurality of inflation lumens are not disposed.

The present invention has been made in view of the above circumstances, and an object of the present invention is to provide a balloon catheter having a first balloon, a second balloon, a first inflation tube having a lumen in communication with a lumen of the first balloon, and a second inflation tube having a lumen in communication with a lumen of the second balloon, the balloon catheter making it less likely for a kink to be generated even when being inserted into a bent portion of a blood vessel.

### SOLUTIONS TO THE PROBLEMS

The present invention is as follows.
[1] A balloon catheter including:
   a shaft extending in a longitudinal direction from a proximal side to a distal side;
   a first balloon and a second balloon each disposed at a distal part of the shaft; and
   a first inflation tube and a second inflation tube each disposed in a lumen of the shaft,
   in which
   the first inflation tube has a lumen in communication with the lumen of the shaft and a lumen of the first balloon,
   the second inflation tube has a lumen in communication with the lumen of the shaft and a lumen of the second balloon,
   the shaft has
   a first section which is present on a distal side and in which the first inflation tube and the second inflation tube are disposed and
   a second section which is present proximal to the first section and in which the first inflation tube and the second inflation tube are disposed,
   at least two of the shaft, the first inflation tube, and the second inflation tube are fixed to each other in the first section, and
   the shaft, the first inflation tube, and the second inflation tube are not fixed to each other in the second section.
[2] The balloon catheter according to [1],
   in which a length W2 of the second section in the longitudinal direction is longer than a length W1 of the first section in the longitudinal direction.
[3] The balloon catheter according to [2],
   in which a ratio (W2/W1) of the length W2 of the second section in the longitudinal direction to the length W1 of the first section in the longitudinal direction has a value of from 1.1 to 2.
[4] The balloon catheter according to any one of [1] to [3],
   in which the shaft further has, in the lumen thereof, a guidewire tube through which a guidewire is inserted, and
   the guidewire tube is partially disposed in the lumen of the first balloon.
[5] The balloon catheter according to any one of [1] to [4],
   in which the first inflation tube has, in the second section, a side wall in which a hole is formed, the hole allowing the lumen of the first inflation tube and the lumen of the shaft to be in communication with each other.
[6] The balloon catheter according to any one of [1] to [5],
   in which the second balloon comprises a plurality of second balloons, and
   the second balloons are arranged in a circumferential direction of an outer periphery of the first balloon.
[7] The balloon catheter according to [6],
   in which the second inflation tube comprises a plurality of second inflation tubes, and
   the first inflation tube has, in the second section, a side wall in which a hole is formed, the hole allowing the lumen of the first inflation tube and the lumen of the shaft to be in communication with each other, or
   at least one of the plurality of second inflation tubes has, in the second section, a side wall in which a hole is formed, the hole allowing the lumen of the at least one second inflation tube and the lumen of the shaft to be in communication with each other.

### ADVANTAGEOUS EFFECTS OF THE INVENTION

The balloon catheter of the present invention has a section in which the first inflation tube and the second inflation tube are disposed inside the lumen of the shaft. In this section, a section in which at least two of the shaft, the first inflation tube, and the second inflation tube are fixed to each other and a section in which the shaft, the first inflation tube, and the second inflation tube are not fixed to each other are present. Since these two sections are present, the difference between rigidities in the longitudinal direction of the shaft can be decreased, whereby it becomes less likely for a kink to be generated even when the balloon catheter of the present invention is inserted into a bent portion of a blood vessel.

### BRIEF DESCRIPTION OF THE DRAWINGS

[FIG. 1] FIG. 1 is a side view (partially transparent view) of a balloon catheter.
[FIG. 2] FIG. 2 is a cross-sectional view in the longitudinal direction of the shaft of the balloon catheter.
[FIG. 3] FIG. 3 is a cross-sectional view in the longitudinal direction of another shaft of the balloon catheter.
[FIG. 4] FIG. 4 is a cross-sectional view in the longitudinal direction of another shaft of the balloon catheter.
[FIG. 5] FIG. 5 is a side view (partially transparent view) of balloons of a balloon catheter.
[FIG. 6] FIG. 6 is a cross-sectional view taken along line I-I of balloons shown in FIG. 5.
[FIG. 7] FIG. 7 is a cross-sectional view of balloons different from balloons shown in FIG. 5.
[FIG. 8] FIG. 8 is a cross-sectional view of balloons different from balloons shown in FIG. 5.

### DESCRIPTION OF EMBODIMENTS

The balloon catheter according to the present invention includes a shaft extending in a longitudinal direction from a proximal side to a distal side, a first balloon and a second balloon each disposed at a distal part of the shaft, and a first inflation tube and a second inflation tube each disposed in a lumen of the shaft, in which the first inflation tube has a lumen in communication with the lumen of the shaft and a lumen of the first balloon, the second inflation tube has a lumen in communication with the lumen of the shaft and a lumen of the second balloon, the shaft has a first section which is present on a distal side and in which the first inflation tube and the second inflation tube are disposed and a second section which is present proximal to the first section and in which the first inflation tube and the second inflation tube are disposed, at least two of the shaft, the first inflation tube, and the second inflation tube are fixed to each other in the first section, and the shaft, the first inflation tube, and the second inflation tube are not fixed to each other in the second section.

The following description specifically explains the balloon catheter according to one or more embodiments of the present invention with reference to the drawings. However, the present invention is not limited to the following embodiments. It is obvious that the present invention can be carried out by making modifications in accordance with the gist described above or later, and such modifications are also included in the technical scope of the present invention. In each drawing, hatching, reference signs for components, and the like may be omitted for convenience of description, and in such a case, the specification and other drawings are to be referred to. The dimensions of the various components in the drawings are provided for the purpose of facilitating the understanding of the feature of the present invention, and the dimensions may differ from the actual dimensions in some cases.

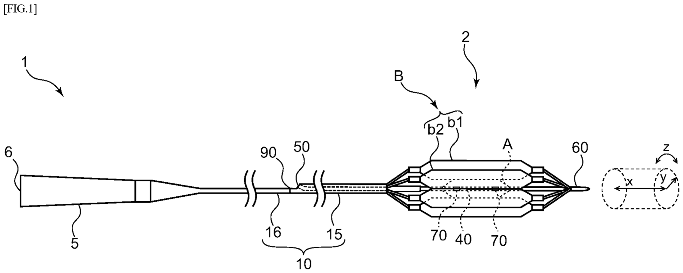

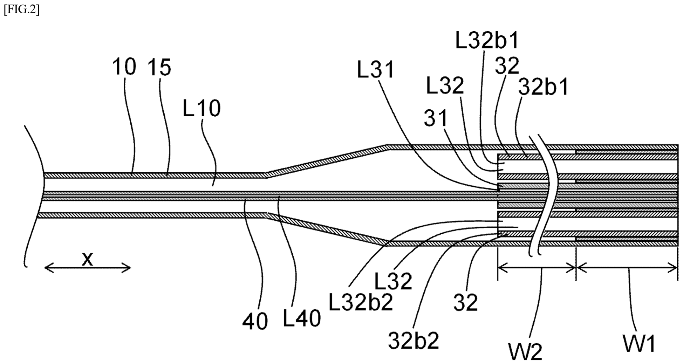

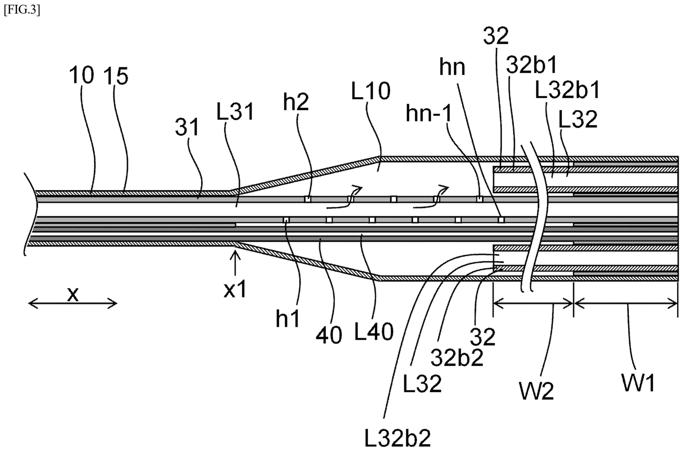

FIG. 1 is a side view (partially transparent view) of the balloon catheter according to the embodiment of the present invention. FIG. 2 is a cross-sectional view in the longitudinal direction of the shaft as a part of the balloon catheter according to the embodiment of the present invention. In each of FIG. 1 and FIG. 2, the left side of the drawing is the proximal side (operator side), and the right side of the drawing is the distal side (lesion side). The same applies also to FIG. 3 to FIG. 5 described below.

As shown in FIG. 1, a balloon catheter 1 according to the embodiment of the present invention has: a shaft 10 extending in the longitudinal direction from the proximal side to the distal side; and a first balloon A and a second balloon B each disposed at a distal part of the shaft 10. In the present description, the first balloon A and the second balloon B are sometimes collectively referred to as "balloon 2".

Each of the number of the first balloons A and the number of the second balloons B may be one. In this case, the first balloon A and the second balloon B may be arranged at the same position in the longitudinal direction or may be arranged on the distal side and the proximal side in the longitudinal direction. In the case of the arrangement on the distal side and the proximal side, the first balloon A and the second balloon B may be respectively disposed on the distal side and the proximal side, or the first balloon A and the second balloon B may be respectively disposed on the proximal side and the distal side.

The second balloon B is preferably composed of a plurality of second balloons B. As shown in FIG. 1, the plurality of second balloons B are more preferably arranged in a circumferential direction of an outer periphery of the first balloon A. In the present description, the plurality of second balloons B are sometimes referred to as "balloon group".

The shaft 10 has: a longitudinal direction x; a radial direction y connecting the centroid of the outer edge of the shaft 10 and any point on the outer edge in a cross section perpendicular to the longitudinal direction x; and a circumferential direction z extending along the outer edge of the shaft 10 in the cross section perpendicular to the longitudinal direction x. In the present description, the hand side of a user in the longitudinal direction x is referred to as "proximal side", and a side opposite to the proximal side, i.e., patient side, is referred to as "distal side".

Each of the members and portions other than the shaft 10 also has a longitudinal direction, a radial direction, and a circumferential direction. These directions may be identical to or different from the longitudinal direction x, the radial direction y, and the circumferential direction z of the shaft 10. However, in the present description, explanations will be given on the assumption that all of the members and portions have a longitudinal direction, a radial direction, and a circumferential direction identical to the longitudinal direction x, the radial direction y, and the circumferential direction z of the shaft 10 in order to facilitate understanding.

The first balloon A and the second balloon B are connected to the distal part of the shaft 10. Introduction of a fluid through a lumen of the shaft 10 enables inflation of the first balloon A and the second balloon B. Meanwhile, discharge of the introduced fluid enables deflation of the first balloon A and the second balloon B. In order to control inflation and deflation of the first balloon A and the second balloon B, an indeflator (balloon pressurizer) may be used to introduce or discharge the fluid. As the fluid, for example, a mixture of a contrast medium and a physiological saline is used. The fluid may be a pressurized fluid obtained through pressurization by a pump or the like.

As shown in FIG. 2, the balloon catheter 1 has a first inflation tube 31 and second inflation tubes 32 (32b1 and 32b2) each disposed in a lumen of the shaft 10. The number of the first inflation tubes 31 only has to be equal to the number of the first balloons A, and the number of the second inflation tubes 32 only has to be equal to the number of the second balloons B. FIG. 2 shows one such first inflation tube 31 and two such second inflation tubes 32b1 and 32b2 in the lumen of the shaft 10. The first inflation tube 31 has a lumen L31, the second inflation tube 32b1 has a lumen L32b1, and the second inflation tube 32b2 has a lumen L32b2.

As shown in FIG. 2, the shaft 10 of the balloon catheter 1 may further have, in the lumen L31 of the first inflation tube 31, a guidewire tube 40 which extends in the longitudinal direction x and through which a guidewire is inserted. The guidewire tube 40 has a guidewire lumen L40. The guidewire tube 40 is preferably partially disposed in a lumen of the first balloon A. Although FIG. 2 shows a configuration example of a coaxial structure in which the guidewire tube 40 is disposed in the lumen L31 of the first inflation tube 31, the guidewire tube 40 and the first inflation tube 31 may be biaxially disposed.

The lumen L31 of the first inflation tube 31 is in communication with a lumen L10 of the shaft 10 and the lumen of the first balloon A (not shown). The lumens L32 (L32b1 and L32b2) of the second inflation tubes 32 (32b1 and 32b2) are in communication with the lumen L10 of the shaft 10 and lumens of the second balloons B (not shown). The first inflation tube 31 and the first balloon A are preferably directly connected to each other. The second inflation tubes 32 (32b1 and 32b2) are preferably directly connected to the respective second balloons B (not shown).

The shaft 10 has: a first section which is present on the distal side and in which the first inflation tube 31 and the second inflation tubes 32 are disposed; and a second section which is present proximal to the first section and in which the first inflation tube 31 and the second inflation tubes 32 are disposed. At least two of the shaft 10, the first inflation tube 31, and the second inflation tubes 32 are fixed to each other in the first section, and the shaft 10, the first inflation tube 31, and the second inflation tubes 32 are not fixed to each other in the second section. Since the shaft 10, the first inflation tube 31, and the second inflation tubes 32 are not fixed to each other in the second section, the second section has a rigidity lower than a rigidity of the first section. Thus, provision of the second section proximal to the first section enables decrease in the difference between the rigidities and makes it less likely for a kink to be generated even when the balloon catheter 1 is inserted into a bent portion of a blood vessel. That is, provision of the second section to the shaft 10 causes the second section to function as a cushion and enables prevention of generation of a kink even when bending force is applied from any side in the radial direction y of the shaft 10. In a case where the shaft 10 has the second section, when the shaft 10 receives such a force as to be pulled so that the first section and the second section are pulled, a portion of the first section near the second section is stretched. In this case as well, in the second section, since the shaft 10, the first inflation tube 31, and the second inflation tubes 32 are not fixed to each other, the first inflation tube 31 and the second inflation tubes 32 are not stretched. As a result, the lumens of the first inflation tube 31 and the second inflation tubes 32 are prevented from being closed. In addition, in a case where, for example, the guidewire tube 40 is further disposed in the lumen L10 of the shaft 10, even when a guidewire inserted into the guidewire tube 40 is brought into contact with a stenosis site or the like and forces from the operator side are concentrated on one location so that the guidewire is pressed outward from inside the guidewire tube 40, the presence of the second section causes the second section to function as a cushion and enables prevention of a kink of the guidewire tube 40.

In FIG. 2, in the first section, the shaft 10 and the second inflation tubes 31 are fixed to each other, and the first inflation tube 31 and the second inflation tubes 32 are fixed to each other. In a case where each of the number of the first inflation tubes 31 and the number of the second inflation tubes 32 is one, the shaft 10 and the first inflation tube 31, the shaft 10 and the second inflation tube 32, and the first inflation tube 31 and the second inflation tube 32 only have to be fixed to each other.

A length W1 of the first section in the longitudinal direction x is, for example, preferably from 8 to 20 mm. The length W1 is more preferably 9 mm or more and further preferably 10 mm or more, and meanwhile, is more preferably 19 mm or less and further preferably 18 mm or less. A length W2 of the second section in the longitudinal direction x is, for example, preferably from 15 to 25 mm. The length W2 is more preferably 16 mm or more and further preferably 17 mm or more, and meanwhile, is more preferably 24 mm or less and further preferably 23 mm or less.

The length W2 of the second section in the longitudinal direction x may be shorter than the length W1 of the first section in the longitudinal direction x (W2<W1) or equal to the length W1 of the first section in the longitudinal direction x (W2=W1), and is preferably longer than the length W1 of the first section in the longitudinal direction x (W2>W1). By the length W2 of the second section in the longitudinal direction x being longer than the length W1 of the first section, the difference between the rigidities of the sections in which the first inflation tube 31 and the second inflation tubes 32 are disposed becomes small. Consequently, it becomes further less likely for a kink to be generated even when the balloon catheter 1 is inserted into a blood vessel.

In a case where the length W2 of the second section in the longitudinal direction x is longer than the length W1 of the first section in the longitudinal direction x (W2>W1), a ratio (W2/W1) of the length W2 of the second section in the longitudinal direction x to the length W1 of the first section in the longitudinal direction x preferably has a value of, for example, from 1.1 to 2. By the value of the ratio (W2/W1) falling within this range, the difference between the rigidities becomes small. Consequently, it becomes further less likely for a kink to be generated even when the balloon catheter 1 is inserted into a blood vessel. The value of the ratio (W2/W1) is more preferably 1.2 or more and further preferably 1.3 or more, and meanwhile, is more preferably 1.9 or less and further preferably 1.8 or less.

As shown in FIG. 3, in the balloon catheter 1, the first inflation tube 31 may have, in the second section, a side wall in which holes h1 to hn are formed, the holes h1 to hn allowing the lumen of the first inflation tube 31 and the lumen L10 of the shaft 10 to be in communication with each other. n represents a positive integer. Among the holes h1 to hn, the hole h1 is a hole located on the most proximal side in the longitudinal direction x, and the hole hn is a hole located on the most distal side in the longitudinal direction x. As shown in FIG. 3, in a case where the first inflation tube 31 has, in the second section, a side wall in which the holes h1 to hn are formed, the holes h1 to hn allowing the lumen of the first inflation tube 31 and the lumen L10 of the shaft 10 to be in communication with each other, the first inflation tube 31 is preferably fixed to the shaft 10 on the proximal side relative to the most proximal position of the hole h1 located on the most proximal side in the longitudinal direction x among the holes formed in the side wall of the first inflation tube 31. The position of the fixation is defined as a fixation position x1. By introducing a fluid into the first inflation tube 31, the fluid is introduced through the lumen L31 of the first inflation tube 31 into the lumen of the first balloon A. At this time, the fluid partially flows out through the holes h1 to hn formed in the side wall of the first inflation tube 31 into the lumen L10 of the shaft 10. The fluid having flowed out is accumulated in the lumen L10 of the shaft 10, and the accumulated fluid is introduced through the lumens L32 (L32b1 and L32b2) of the second inflation tubes 32 (32b1 and 32b2) into the second balloons B. By thus introducing a fluid into the first inflation tube 31, the order of inflation of the first balloon A and the second balloons B can be controlled. As a result, when the first balloon A and the second balloons B are disposed in a lesion and inflated, the presence of a time interval from inflation of the first balloon A to inflation of the second balloons B enables fine adjustment of the arrangement positions of the first balloon A and the second balloons B, whereby the balloon 2 can be easily positioned. In addition, since the second balloons B are inflated later than the first balloon A, the perfusion rate of blood can be maintained also during the fine adjustment of the arrangement positions of the first balloon A and the second balloons B.

As shown in FIG. 3, the first inflation tube 31 may extend to the hand side such that the shaft 10 covers the outer side of the first inflation tube 31. Alternatively, it is also allowable that: only the first inflation tube 31 extends to the hand side; the shaft 10 has a proximal end part having an inner surface fixed to the outer surface of the first inflation tube 31; and the shaft 10 is disposed only on the distal side from the fixation position x1 between the first inflation tube 31 and the shaft 10. Alternatively, it is also allowable that: only the shaft 10 extends to the hand side; the first inflation tube 31 has a proximal end part having an outer surface fixed to the inner surface of the shaft 10; and the first inflation tube 31 is disposed only on the distal side from the fixation position x1 between the first inflation tube 31 and the shaft 10. By using one of the first inflation tube 31 or the shaft 10 as a member extending to the hand side, the outer diameter of the balloon catheter 1 can be decreased, whereby minimal invasiveness can be increased. The first inflation tube 31 and the shaft 10 are preferably fixed to each other by, for example, using an adhesive or performing thermal welding.

In a case where the second inflation tubes 32 (32b1 and 32b2) are disposed in the lumen L10 of the shaft 10, the number of the second inflation tubes 32 (32b1 and 32b2) only has to be set according to the number of the second balloons B. That is, although FIG. 3 shows a configuration example in which the second inflation tube 32 present in the lumen L10 of the shaft 10 is composed of a plurality of second inflation tubes 32, the number of the second inflation tubes 32 may be one if the number of the second balloons B is one.

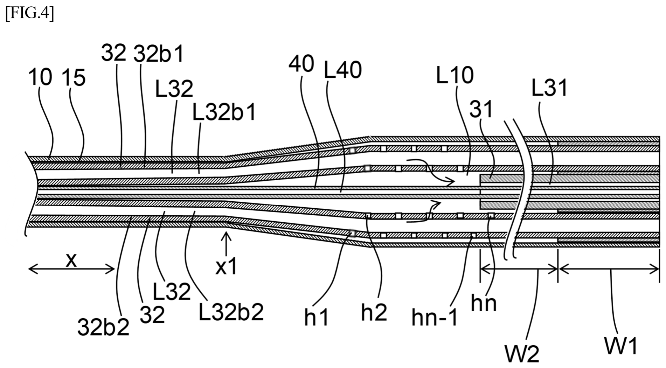

In the balloon catheter 1, in a case where the second inflation tube 32 present in the lumen L10 of the shaft 10 is composed of a plurality of second inflation tubes 32, the first inflation tube 31 may have, in the second section, a side wall in which holes are formed, the holes allowing the lumen L31 of the first inflation tube 31 and the lumen L10 of the shaft 10 to be in communication with each other as described above. Alternatively, as shown in FIG. 4, at least one of the plurality of second inflation tubes 32 may have, in the second section, a side wall in which holes are formed, the holes allowing the lumen L32 of the at least one second inflation tube 32 and the lumen L10 of the shaft 10 to be in communication with each other. In a case where the at least one second inflation tube 32 has, in the second section, a side wall in which holes are formed, the holes allowing the lumen L32 of the at least one second inflation tube 32 and the lumen L10 of the shaft 10 to be in communication with each other, the at least one second inflation tube 32 is preferably fixed to the shaft 10 on the proximal side relative to the most proximal position of the hole h1 located on the most proximal side in the longitudinal direction x among the holes formed in the side wall of the at least one second inflation tube 32. The position of the fixation is defined as a fixation position x1. By introducing a fluid into the second inflation tubes 32 (32b1 and 32b2), the fluid is introduced through the lumens L32 (L32b1 and L32b2) of the second inflation tubes 32 (32b1 and 32b2) into the lumens of the second balloons B. At this time, the fluid partially flows out through the holes h1 to hn formed in the side wall of the at least one of the second inflation tubes 32 (32b1 and 32b2) into the lumen L10 of the shaft 10. The fluid having flowed out is accumulated in the lumen L10 of the shaft 10, and the accumulated fluid is introduced through the lumen L31 of the first inflation tube 31 into the first balloon A. By thus introducing a fluid into the second inflation tubes 32, the order of inflation of the first balloon A and the second balloons B can be controlled. As a result, since the first balloon A is inflated later than the second balloons B, the first balloon A causes expansion, in the radial direction y, of the plurality of second balloons B having been inflated first, whereby the extent of close contact between the second balloons B and the inner wall of the blood vessel becomes large.

As shown in FIG. 4, each of the second inflation tubes 32 may extend to the hand side such that the shaft 10 covers the outer side of the second inflation tube 32. Alternatively, it is also allowable that: only the second inflation tube 32 extends to the hand side; the shaft 10 has a proximal end part having an inner surface fixed to the outer surface of the second inflation tube 32; and the shaft 10 is disposed only on the distal side from the fixation position x1 between the second inflation tube 32 and the shaft 10. Alternatively, it is also allowable that: only the shaft 10 extends to the hand side; the second inflation tube 32 has a proximal end part having an outer surface fixed to the inner surface of the shaft 10; and the second inflation tube 32 is disposed only on the distal side from the fixation position x1 between the second inflation tube 32 and the shaft 10. By using one of the second inflation tube 32 or the shaft 10 as a member extending to the hand side, the outer diameter of the balloon catheter 1 can be decreased, whereby minimal invasiveness can be increased. The second inflation tube 32 and the shaft 10 are preferably fixed to each other by, for example, using an adhesive or performing thermal welding.

In a case where at least one of the plurality of second inflation tubes 32 has, in the second section, a side wall in which holes are formed, the holes allowing the lumen L32 of the at least one second inflation tube 32 and the lumen L10 of the shaft 10 to be in communication with each other, the holes may be formed in each of the plurality of second inflation tubes 32 (32b1 and 32b2) as shown in FIG. 4 or may be formed in only one of the plurality of second inflation tubes 32.

The number of the holes formed in the side wall of the first inflation tube 31 or the at least one second inflation tube 32 only has to be one or more and only has to be set as appropriate based on, for example, the time difference between inflation of the first balloon A and inflation of the second balloons B. The number of the holes is, for example, preferably 5 or more and more preferably 10 or more. Regarding the upper limit of the number of the holes, the number is, for example, preferably 40 or less, more preferably 35 or less, and further preferably 30 or less.

The positions of the holes formed in the side wall of the first inflation tube 31 or the at least one second inflation tube 32 are preferably set such that a fluid introduced into the first inflation tube 31 or the at least one second inflation tube 32 is evenly discharged from the inner side to the outer side in the radial direction y of the first inflation tube 31 or the at least one second inflation tube 32. Specifically, the holes may be arranged side-by-side in the longitudinal direction x, spirally arranged with respect to the longitudinal direction x, or randomly arranged.

The sizes of the holes formed in the side wall of the first inflation tube 31 or the at least one second inflation tube 32 only have to be set as appropriate based on, for example, the time difference between inflation of the first balloon A and inflation of the second balloons B.

As shown in FIG. 2 to FIG. 4, the shaft 10 of the balloon catheter 1 may further have the guidewire tube 40 which extends in the longitudinal direction x and through which a guidewire is inserted. In a case where the guidewire tube 40 is provided, the first inflation tube 31 and the second inflation tubes 32 are preferably disposed around the outer periphery of the guidewire tube 40 as shown in FIG. 3. Consequently, the first inflation tube 31 and the second inflation tubes 32 support the guidewire tube 40, and thus damage to the guidewire tube 40 can be prevented. As shown in FIG. 2 and FIG. 4, the guidewire tube 40 is preferably partially disposed in the lumen L31 of the first inflation tube 31. By the guidewire tube 40 being partially disposed in the lumen L31 of the first inflation tube 31, operability of the balloon catheter 1 becomes favorable. The guidewire tube 40 is preferably partially disposed in the lumen of the first balloon A. Consequently, damage to the guidewire tube 40 can be further prevented. The guidewire tube 40 may be disposed in the lumen L10 of the shaft 10 parallelly to the first inflation tube 31 to obtain a biaxial configuration as shown in FIG. 3 or may be disposed in the lumen L31 of the first inflation tube 31 to obtain a coaxial structure as shown in FIG. 2 and FIG. 4.

Examples of a material forming the shaft 10 include polyamide-based resins, polyester-based resins, polyurethane-based resins, polyolefin-based resins, fluorine-based resins, vinyl chloride-based resins, silicone-based resins, and natural rubbers. These types of materials may be used singly, or two or more of these types of materials may be used in combination. Among these materials, at least one material selected from polyamide-based resins, polyolefin-based resins, and fluorine-based resins is preferable as the material forming the shaft 10. By the material being any of polyamide-based resins, polyolefin-based resins, or fluorine-based resins, the slipperiness of the surface of the shaft 10 becomes high, whereby the insertability of the balloon catheter 1 in a blood vessel can be improved.

Examples of a material forming the first inflation tube 31, a material forming the second inflation tubes 32, and a material forming the guidewire tube 40 include polyamide-based resins, polyester-based resins, polyurethane-based resins, polyolefin-based resins, fluorine-based resins, vinyl chloride-based resins, silicone-based resins, and natural rubbers. These types of materials may be used singly, or two or more of these types of materials may be used in combination. Among these materials, at least one material selected from polyamide-based resins, polyolefin-based resins, and fluorine-based resins is preferable. The material forming the first inflation tube 31, the material forming the second inflation tubes 32, and the material forming the guidewire tube 40 may be different from one another but are preferably identical to one another. By using identical materials, the rigidity can be made even.

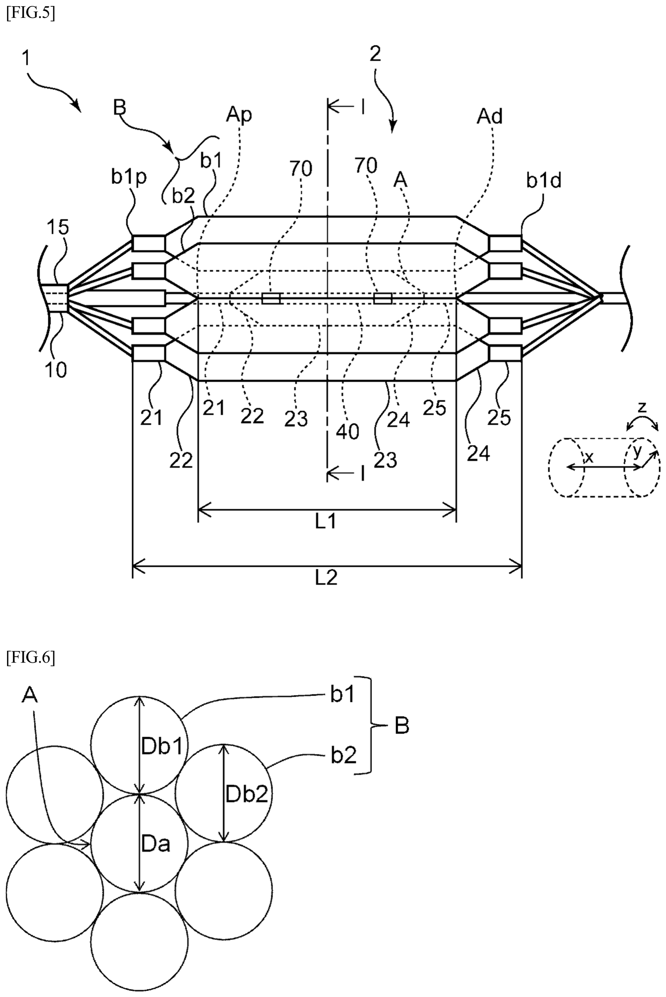

As shown in FIG. 1, the balloon catheter 1 is preferably such that: the second balloon B is composed of a plurality of second balloons B; and the plurality of second balloons B are arranged in the circumferential direction z of the outer periphery of the first balloon A. Arrangement of the first balloon A and the second balloons B will be described with reference to FIG. 5 and the like.

FIG. 5 is a side view (partially transparent view) of the balloons of the balloon catheter 1. FIG. 5 shows a state where the first balloon A and a balloon group including the plurality of second balloons B are inflated. FIG. 6 is a I-I cross-sectional view of the balloon 2 shown in FIG. 5. The balloon catheter 1 shown in FIG. 5 and FIG. 6 includes one first balloon A and six second balloons B, and the six second balloons B constitute a balloon group. In FIG. 5 and FIG. 6, two second balloons B among the six second balloons B are denoted by the reference character b1 and the reference character b2 for convenience in explanations. During inflation of the first balloon A and the second balloons B (b1 and b2), the first balloon A has a maximum outer diameter denoted by Da, and the second balloons b1 and b2 have maximum outer diameters denoted by Db1 and Db2, respectively.

As shown in FIG. 5 and FIG. 6, in the balloon catheter 1, the balloon group including the plurality of second balloons B is arranged in the circumferential direction z of the outer periphery of the first balloon A, and inflation of both the first balloon A and the balloon group enables increase in the outer diameter of the balloon 2. As a result, the stenosis site can be assuredly dilated.

As shown in FIG. 5 and FIG. 6, the balloon catheter 1 is preferably configured such that, during inflation of the first balloon A and the balloon group, the adjacent second balloon b1 and second balloon b2 among the plurality of second balloons B constituting the balloon group are in contact with each other. By the adjacent second balloon b1 and second balloon b2 being in contact with each other, the second balloons mutually suppress respective inflations. Consequently, a high withstanding pressure is obtained, whereby expansive force of the balloon group can be increased. In addition, by the adjacent second balloon b1 and second balloon b2 being in contact with each other, the second balloons B become less likely to be displaced in the circumferential direction z of the outer periphery of the first balloon A even when the second balloons B are brought into contact with a stenosis site. Consequently, the stenosis site can be assuredly dilated.

During inflation of the first balloon A and the balloon group , only at least one pair of adjacent second balloons among the plurality of second balloons B constituting the balloon group have to be in contact with each other, two or more pairs of adjacent second balloons B among the plurality of second balloons B are preferably in contact with each other, and all the adjacent second balloons B among the plurality of second balloons B are more preferably in contact with each other. When all the adjacent second balloons B are in contact with each other, the second balloons B can be evenly inflated.

During inflation of the first balloon A and the balloon group, the second balloons B constituting the balloon group do not have to be in contact with an outer peripheral surface of the first balloon A, but at least one of the plurality of second balloons B constituting the balloon group is preferably in contact with the outer peripheral surface of the first balloon A. By at least one of the plurality of second balloons B being in contact with the outer peripheral surface of the first balloon A, the at least one second balloon B in contact with the outer peripheral surface of the first balloon A makes it less likely for the second balloons B to be displaced in the radial direction y of the first balloon A even when the second balloons B are pressed against a stenosis site. Consequently, the stenosis site can be assuredly dilated. All of the plurality of second balloons B constituting the balloon group are more preferably in contact with the outer peripheral surface of the first balloon A. When all of the plurality of second balloons B are in contact with the outer peripheral surface of the first balloon A, the first balloon A and the second balloons B are mutually inflated even when the second balloons B are pressed against a stenosis site. Consequently, the withstanding pressure of the balloon group can be increased.

The number of the second balloons B constituting the balloon group is preferably 3 or more, more preferably 4 or more, and further preferably 5 or more. By setting the lower limit value of the number of the second balloons B constituting the balloon group to the above range, the balloon group easily encloses the outer periphery of the first balloon A and easily suppresses inflation of the first balloon A. As a result, when a fluid is introduced into both the first balloon A and the second balloon B, the first balloon A and the second balloon B mutually suppress respective inflations. Consequently, the withstanding pressure of the balloon 2 can be increased, and the hardness of the balloon 2 is increased, whereby expansive force of the balloon 2 can be improved. In addition, since the first balloon A and the balloon group mutually suppress respective inflations, the balloon 2 becomes less likely to be inflated. Consequently, the balloon 2 is inhibited from being excessively inflated even when a high pressure is applied to the balloon 2. Thus, the balloon 2 is prevented from being inflated to an outer diameter larger than an intended outer diameter, and damage to a lumen inside a living organism such as the aortic valve is decreased, whereby safety can be improved. The upper limit of the number of the second balloons B constituting the balloon group is not particularly limited. However, for example, the number is preferably 20 or less, more preferably 12 or less, further preferably 10 or less, and particularly preferably eight or less. By setting the upper limit value of the number of the second balloons B constituting the balloon group to the above range, the second balloons B constituting the balloon group become less likely to be displaced in the radial direction y and the circumferential direction z of the first balloon A, and it becomes easy for the balloon group to suppress inflation of the first balloon A.

During inflation of the first balloon A and the balloon group, a maximum outer diameter of a circumcircle of the balloon 2 is not particularly limited but is, for example, preferably within a range of 5 mm to 60 mm.

The relationship between the maximum outer diameter of the first balloon A and the maximum outer diameters of the second balloons B constituting the balloon group is preferably any one of the following (1) to (3), for example.
(1) During inflation of the first balloon A and the balloon group, magnitudes of the maximum outer diameters of the second balloons B constituting the balloon group are all equal to one another and are equal to a magnitude of the maximum outer diameter of the first balloon A.
(2) During inflation of the balloon group, the second balloons B constituting the balloon group are two or more types of second balloons B having respective different magnitudes of maximum outer diameters.
(3) During inflation of the first balloon A and the balloon group, the magnitudes of the maximum outer diameters of the second balloons B constituting the balloon group are all equal to one another and are different from the magnitude of the maximum outer diameter of the first balloon A.

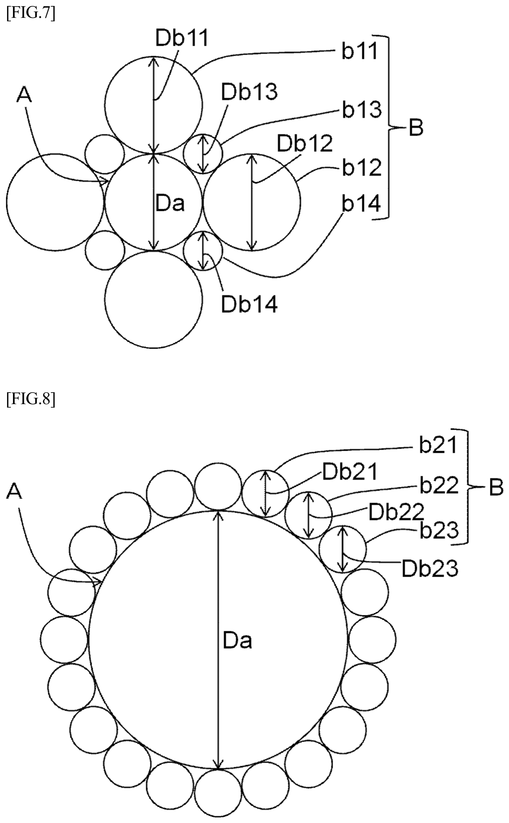

The maximum outer diameter of the first balloon A refers to the maximum equivalent circular diameter of the first balloon A in a cross section perpendicular to the longitudinal direction x of the first balloon A. The maximum outer diameter of each second balloon B refers to the maximum equivalent circular diameter of the second balloon B in a cross section perpendicular to the longitudinal direction x of the second balloon B.
(1) will be described with reference to FIG. 6. As shown in FIG. 6, during inflation of the first balloon A and the balloon group, the magnitudes of the maximum outer diameters of the second balloons B constituting the balloon group are all equal to one another and are equal to the magnitude of the maximum outer diameter of the first balloon A. Consequently, balance is easily attained between a force of inflation of the first balloon A and forces of inflation of the second balloons B to suppress the inflation of the first balloon A. As a result, the hardnesses of the first balloon A and the balloon group are increased, the expansive force of the balloon group can be easily increased, and the stenosis site can be evenly dilated.
   In a case where the maximum outer diameters of the first balloon A and all of the second balloons B are equal to one another, the maximum outer diameters of the first balloon A and the second balloons B are, for example, preferably from 3 mm to 8 mm. The magnitudes of the maximum outer diameters of the plurality of second balloons B constituting the balloon group being all equal to one another means that the maximum outer diameters of the plurality of second balloons B constituting the balloon group are approximately equal to one another, and specifically means that the largest maximum outer diameter among the maximum outer diameters of the second balloons B constituting the balloon group is 100% or more and 110% or less of the smallest maximum outer diameter among the maximum outer diameters of the second balloons B constituting the balloon group. The maximum outer diameter of the first balloon A being equal to the maximum outer diameters of the plurality of second balloons B constituting the balloon group means that the maximum outer diameter of the first balloon A and the maximum outer diameters of the plurality of second balloons B constituting the balloon group are approximately equal to one another, and specifically means that the maximum outer diameter of the first balloon A is 90% or more and 110% or less of a maximum outer diameter (e.g., average value) of the second balloons B.
(2) will be described with reference to FIG. 7. FIG. 7 is a cross-sectional view of balloons different from the balloons shown in FIG. 5, at a position corresponding to the position in FIG. 6. As shown in FIG. 7, during inflation of the balloon group, the second balloons B constituting the balloon group are two or more types of second balloons B having respective different magnitudes of maximum outer diameters, whereby the plurality of second balloons having different maximum outer diameters are disposed in the circumferential direction z of the outer periphery of the first balloon A. Consequently, the balloon group includes: second balloons B (b11 and b12) that have a larger maximum outer diameter and that are in contact with the inner wall of the blood vessel; and second balloons B (b13 and b14) that have a smaller maximum outer diameter and that are not in contact with the inner wall of the blood vessel. Thus, spaces are easily formed between the inner wall of the blood vessel and the second balloons B (b13 and b14) that are not in contact with the inner wall of the blood vessel, whereby blood can be perfused through the spaces. In addition, since the second balloons B (b11 and b12) having the larger maximum outer diameter in the balloon group are brought into contact with the inner wall of the blood vessel, the number of the second balloons B that are brought into contact with the inner wall of the blood vessel in the balloon group B is limited. Consequently, the number of the contact points between the second balloons B and the inner wall of the blood vessel becomes small, whereby stresses to be applied from the second balloons B to the inner wall of the blood vessel can be concentrated. As a result, the stenosis site can be assuredly dilated.

In a case where the number of the second balloons constituting the balloon group is at least three, and, during inflation of the first balloon A and the balloon group, two types of the second balloons B having the respective different magnitudes of maximum outer diameters are disposed in the circumferential direction z of the outer periphery of the first balloon A, the two types of second balloons B preferably include the second balloons b11 and b12 having the larger maximum outer diameter and the second balloon b13 that has the smaller maximum outer diameter and that is interposed between the second balloons b11 and b12, as shown in FIG. 7. By such second balloons b13 having the smaller maximum outer diameter being interposed between the second balloons b11 and b12 having the larger maximum outer diameter in the circumferential direction z of the outer periphery of the first balloon A, spaces formed between the inner wall of the blood vessel and the second balloons B that are not in contact with the inner wall of the blood vessel are present in a distributed manner in the circumferential direction z of the outer periphery of the first balloon A. Consequently, blood can be stably perfused.

During inflation of the balloon group, the plurality of second balloons B constituting the balloon group may be two types, three types, or four or more types of second balloons B having respective different magnitudes of maximum outer diameters, for example. In particular, the second balloons B constituting the balloon group are preferably two types of second balloons B having respective different magnitudes of maximum outer diameters.

As shown in FIG. 7, in a case where the plurality of second balloons B constituting the balloon group are two types of second balloons B having respective different magnitudes of maximum outer diameters during inflation of the first balloon A and the balloon group B, a ratio (Db11/Db13) of a maximum outer diameter Db11 of the second balloon b11 as the larger maximum outer diameter to a maximum outer diameter Db13 of the second balloon b13 as the smaller maximum outer diameter is, for example, preferably more than 1 and 5 or less. The ratio (Db11/Db13) is more preferably 1.1 or more, further preferably more than 1.1, particularly preferably 2 or more, and most preferably 2.5 or more, and meanwhile, is more preferably 4.5 or less and further preferably 4 or less.

As shown in FIG. 7, in the case where the plurality of second balloons constituting the balloon group are two types of second balloons having respective different magnitudes of maximum outer diameters during inflation of the first balloon A and the balloon group, the maximum outer diameter Db13 of the second balloon b13 as the smaller maximum outer diameter is, for example, preferably from 3 mm to 5 mm, and the maximum outer diameter Db11 of the second balloon b11 as the larger maximum outer diameter is, for example, preferably from 3.1 mm to 25 mm (in particular, more than 3.3 mm and 25 mm or less).

(3) will be described with reference to FIG. 8. FIG. 8 is a cross-sectional view of balloons different from the balloons shown in FIG. 5, at the position corresponding to the position in FIG. 6. As shown in FIG. 8, during inflation of the first balloon A and the balloon group, the magnitudes of the maximum outer diameters of the plurality of second balloons B constituting the balloon group are all equal to one another but are different from the magnitude of the maximum outer diameter of the first balloon A. Consequently, a first balloon A and second balloons B having different magnitudes of maximum outer diameters can be combined as appropriate to make adjustment, whereby the magnitude of the maximum outer diameter of the balloon group is easily adjusted. The magnitudes of the maximum outer diameters of the plurality of second balloons B constituting the balloon group being all equal to one another means that the maximum outer diameters of the plurality of second balloons B constituting the balloon group are approximately equal to one another, and specifically means that the largest maximum outer diameter among the maximum outer diameters of the second balloons B constituting the balloon group is 100% or more and 110% or less of the smallest maximum outer diameter among the maximum outer diameters of the second balloons B constituting the balloon group.

In the case of (3), during inflation of the first balloon A and the balloon group, the magnitudes of the maximum outer diameters of the plurality of second balloons B constituting the balloon group may be larger or smaller than the magnitude of the maximum outer diameter of the first balloon A, but are preferably smaller than the magnitude of the maximum outer diameter of the first balloon A. By the magnitudes of the maximum outer diameters of the plurality of second balloons B constituting the balloon group being smaller than the magnitude of the maximum outer diameter Da of the first balloon A, the first balloon A can be inflated at a high pressure. Consequently, the stenosis site can be assuredly dilated.

In a case where the magnitudes of the maximum outer diameters of the plurality of second balloons B constituting the balloon group are larger than the magnitude of the maximum outer diameter Da of the first balloon A, a ratio (Db21/Da) of a maximum outer diameter Db21 of a second balloon b21 to the maximum outer diameter Da of the first balloon A is, for example, preferably more than 1 and 4.5 or less. The ratio (Db21/Da) is more preferably 1.1 or more, further preferably more than 1.1, and particularly preferably 1.2 or more, and meanwhile, is more preferably 4 or less and further preferably 3 or less.

In the case where the magnitudes of the maximum outer diameters of the plurality of second balloons B constituting the balloon group are larger than the magnitude of the maximum outer diameter of the first balloon A, the maximum outer diameter Da of the first balloon A is, for example, preferably from 3 mm to 5 mm, and the maximum outer diameter Db21 of the second balloon b21 is, for example, preferably from 3.1 mm to 13.5 mm (in particular, more than 3.3 mm and 13.5 mm or less).

In a case where the magnitudes of the maximum outer diameters of the plurality of second balloons B constituting the balloon group are smaller than the magnitude of the maximum outer diameter of the first balloon A, a ratio (Db21/Da) of a maximum outer diameter Db21 of a second balloon B to the maximum outer diameter Da of the first balloon A is, for example, preferably 0.01 or more and less than 1. The ratio (Db21/Da) is more preferably 0.03 or more, and further preferably 0.05 or more, and meanwhile, is more preferably less than 0.9, further preferably 0.2 or less, and particularly preferably 0.1 or less.

In the case where the magnitudes of the maximum outer diameters of the plurality of second balloons B constituting the balloon group are smaller than the magnitude of the maximum outer diameter Da of the first balloon A, the maximum outer diameter Da of the first balloon A is, for example, preferably from 3 mm to 20 mm, and the maximum outer diameter Db21 of the second balloon b21 is, for example, preferably from 1.0 mm to 5 mm.

As shown in FIG. 5, each of the first balloon A and the second balloons B of the balloon catheter 1 preferably has a straight tubular part 23, a proximal tapered part 22 located proximal to the straight tubular part 23, and a distal tapered part 24 located distal to the straight tubular part 23, and may have a proximal sleeve part 21 located proximal to the proximal tapered part 22 and a distal sleeve part 25 located distal to the distal tapered part 24.

During inflation of the first balloon A and the balloon group, a length L1 of the first balloon A from a distal end Ad thereof to a proximal end Ap thereof in the longitudinal direction x may be equal to a length L2 of the second balloon b1 from a distal end b1d thereof to a proximal end blp thereof in the longitudinal direction x, but is preferably shorter than the length L2. By the length L1 of the first balloon A being shorter than the length L2 of the second balloon b1, a portion at which the first balloon A is present is easily inflated to a larger extent than a portion at which the first balloon A is not present. As a result, at the portion at which the first balloon A is present, pressure is easily applied, whereby pressure can be applied precisely to a target site. Meanwhile, the portion at which the first balloon A is not present is less likely to be inflated to a large extent, whereby pressure can be made less likely to be applied at this portion. Consequently, a load is less likely to be applied to a site other than the target site, whereby the minimal invasiveness of the balloon catheter 1 can be improved.

During inflation of the first balloon A and the balloon group, the length L1 of the first balloon A from the distal end Ad thereof to the proximal end Ap thereof in the longitudinal direction x is preferably 95% or less, more preferably 90% or less, and further preferably 85% or less of the length L2 of the second balloon b1 from the distal end b1d thereof to the proximal end blp thereof in the longitudinal direction x. By setting the upper limit value of the ratio of the length L1 of the first balloon A to the length L2 of the second balloon b1 to the above range, it can be made easy for the balloon catheter 1 to apply a high pressure precisely to the target site. Meanwhile, during inflation of the first balloon A and the balloon group, the length L1 of the first balloon A from the distal end Ad thereof to the proximal end Ap thereof in the longitudinal direction x is preferably 20% or more, more preferably 25% or more, and further preferably 30% or more of the length L2 of the second balloon b1 from the distal end b1d thereof to the proximal end blp thereof in the longitudinal direction x. By setting the lower limit value of the ratio of the length L1 of the first balloon A to the length L2 of the second balloon b1 to the above range, it becomes easy for the balloon catheter 1 to apply pressure to a sufficient range of the target site, whereby dilation of the stenosis site, rupture of a bioprosthetic valve, or the like is easily performed.

As shown in FIG. 1, the type of the balloon catheter 1 can be exemplified by a so-called rapid-exchange type balloon catheter having: a guidewire port 50 located midway between the distal and proximal sides of the shaft 10; and a guidewire insertion path extending from the guidewire port 50 to the distal side of the shaft 10. As shown in FIG. 1, the balloon catheter 1 has a hub 5 on the hand side, and the hub 5 is provided with a fluid inlet 6 into which a fluid for inflating or deflating the balloon 2 is injected.

In the case of a rapid-exchange type balloon catheter such as one shown in FIG. 1, the shaft 10 of the balloon catheter 1 preferably has a distal shaft 15 and a proximal shaft 16 disposed proximal to the distal shaft 15, and the distal shaft 15 and the proximal shaft 16 may be separate members. In a case where the distal shaft 15 and the proximal shaft 16 are separate members, the proximal shaft 16 may be made from a resin or a metal.

In the case of a rapid-exchange type balloon catheter such as one shown in FIG. 1, the proximal shaft 16 and/or the distal shaft 15 preferably has an outer wall provided with a coating, and each of both the proximal shaft 16 and the distal shaft 15 is more preferably provided with a coating. The coating may be a hydrophilic coating or a hydrophobic coating according to purpose and can be provided by: immersing the shaft 10 in a hydrophilic coating agent or a hydrophobic coating agent; applying the hydrophilic coating agent or the hydrophobic coating agent onto the outer wall of the shaft 10; or coating the outer wall of the shaft 10 with the hydrophilic coating agent or the hydrophobic coating agent. Each of the coating agents may contain a drug and an additive.

Examples of the hydrophilic coating agent include: hydrophilic polymers such as polyvinyl alcohol, polyethylene glycol, polyacrylamide, polyvinylpyrrolidone, and methyl vinyl ether-maleic anhydride copolymers; and hydrophilic coating agents made by any combination of these hydrophilic polymers. Examples of the hydrophobic coating agent include polytetrafluoroethylene (PTFE), fluorinated ethylene propylene (FEP), perfluoroalkoxy alkane (PFA), silicone oil, hydrophobic urethane resins, carbon coats, diamond coats, diamond-like carbon (DLC) coats, ceramic coats, and substances with low surface free energy terminated with an alkyl group or a perfluoro alkyl group.

The type of the balloon catheter 1 may be a so-called over-the-wire type balloon catheter (not shown) having a guidewire insertion path extending from the distal side to the proximal side of the shaft. In the case of the over-the-wire type balloon catheter, the inflation lumens and the guidewire lumen preferably extend to the hub 5 disposed on the hand side, and each of the lumens preferably has a proximal opening provided in the hub 5 having a bifurcated structure. In the case of the over-the-wire type balloon catheter, an outer shaft preferably has an outer wall provided with a coating. For a material forming the shaft and a coating thereon, description of the rapid-exchange type balloon catheter may be referred to.

Examples of a material forming the first balloon A and materials forming the second balloons B include: polyolefin-based resins such as polyethylene, polypropylene, and ethylene-propylene copolymers; polyester-based resins such as polyethylene terephthalate and polyester elastomers; polyurethane-based resins such as polyurethane and polyurethane elastomers; polyphenylene sulfide-based resins; polyamide-based resins such as polyamides and polyamide elastomers; fluorine-based resins; silicone-based resins; and natural rubbers such as latex rubber. These types of materials may be used singly, or two or more of these types of materials may be used in combination. Among these materials, at least one material selected from polyamide-based resins, polyester-based resins, and polyurethane-based resins is preferable as each of the material forming the first balloon A and the materials forming the second balloons B.

As each of the material forming the first balloon A and the materials forming the second balloons B, an elastomer resin is preferably used from the viewpoint of film thinning and flexibility. For example, among the polyamide-based resins, nylon 12, nylon 11, or the like is suitable as each of the material forming the first balloon A and the materials forming the second balloons B. Nylon 12 is more suitable since nylon 12 can be comparatively easily molded during blow molding. From the viewpoint of film thinning and flexibilities of the first balloon A and the second balloons B, any of polyamide elastomers such as polyether ester amide elastomers and polyamide ether elastomers is preferably used. Among these polyamide elastomers, any of polyether ester amide elastomers is preferably used since polyether ester amide elastomers have high yield strengths and allow the first balloon A and the second balloons B to have favorable dimensional stabilities.

The material forming the first balloon A and the materials forming the second balloons B may be identical to each other but are preferably different from each other. The materials are preferably selected such that the withstanding pressure value P2 of the second balloons B becomes larger than the withstanding pressure value P1 of the first balloon A. The materials forming the plurality of second balloons B may be different from one another but are preferably identical to one another. By the materials being identical to one another, the extents of inflation, the hardnesses, and the like of the individual second balloons B can be set to be approximately equal to one another.

The shaft 10 disposed inside the first balloon A has a portion corresponding to the position of the first balloon A in the longitudinal direction x. On this portion, a radiopaque marker 70 may be disposed such that the position of the first balloon A can be ascertained radiographically. The radiopaque marker 70 is preferably disposed at each of positions corresponding to both ends of the straight tubular part 23 of the first balloon A and may be disposed at a position corresponding to the center of the straight tubular part 23 of the first balloon A. The shape of the radiopaque marker 70 is preferably a tubular shape. Examples of the tubular shape include a cylindrical shape, a polygonal tubular shape, a shape obtained by forming a slit in a tube so as to have a C-shaped cross section, and a coil shape obtained by winding a wire material. As a material forming the radiopaque marker 70, for example, a radiopaque substance such as lead, barium, iodine, tungsten, gold, platinum, iridium, stainless steel, titanium, or a cobalt-chromium alloy may be used.

The balloon catheter 1 is preferably provided with a tip member 60 at a distal end part thereof. The tip member 60 may be provided to the distal end part of the balloon catheter 1 by being connected to a distal end part of the first balloon A as a member separate from the shaft 10, or a distal end part of the shaft 10 may function as the tip member 60 by the shaft 10 extending to a position distal to the distal end of the first balloon A.

The balloon catheter 1 is usable for, for example, dilation of a blood vessel. In addition, the balloon catheter 1 is usable also for, for example, dilation of an aortic valve, deformation of a bioprosthetic valve implanted in a heart, or rupture of the bioprosthetic valve. A stenosis of a blood vessel occurs also in, for example, the aortic valve. In a case where a stenosis occurs in the aortic valve, the aortic valve is removed, and a new bioprosthetic valve is implanted. However, the implanted bioprosthetic valve degrades over time and needs to be replaced after about 5 to 10 years. In a case where treatment for transcatheter implantation of a new bioprosthetic valve (prosthetic valve) is conducted to deal with degradation of the surgically implanted bioprosthetic valve (prosthetic valve), the area of the valve port sometimes becomes small. Thus, the size of the new bioprosthetic valve needs to be smaller than the bioprosthetic valve having degraded. This decreased size leads to decrease in the flow rate of blood and occurrence of a pressure difference between the front and rear sides of the bioprosthetic valve, resulting in a burden on the heart. In view of this, the bioprosthetic valve having been implanted in the heart is deformed or ruptured using the balloon catheter 1. By doing so, a larger bioprosthetic valve can be implanted, and the pressure difference between the front and rear sides of the bioprosthetic valve can be improved.

A method for manufacturing the balloon catheter 1 is not particularly limited. For example, the method may include: inserting the first inflation tube 31, the second inflation tubes 32, and, as necessary, the guidewire tube 40 into the lumen L10 of the shaft 10; and achieving fixation therebetween by using an adhesive or performing thermal welding. In this case, the second section in which the shaft 10, the first inflation tube 31, and the second inflation tubes 32 are not fixed to each other is formed proximal to the first section in which at least two of the shaft 10, the first inflation tube 31, and the second inflation tubes 32 are fixed to each other.

This application claims the benefit of the priority date of Japanese patent application No. 2023-102824 filed on June 22, 2023. All of the contents of the Japanese patent application No. 2023-102824 are incorporated by reference herein.

### REFERENCE SIGNS LIST

1 Balloon catheter
2 Balloon
5 Hub
6 Fluid inlet
10 Shaft
15 Distal shaft
16 Proximal shaft
21 Proximal sleeve part
22 Proximal tapered part
23 Straight tubular part
24 Distal tapered part
25 Distal sleeve part
31 First inflation tube
32, 32b1, 32b2 Second inflation tubes
40 Guidewire tube
50 Guidewire port
60 Tip member
70 Radiopaque marker
90 Boundary line between the distal shaft 15 and the proximal shaft 16
A First balloon
B Second balloon
b1, b2 b11 to b14, and b21 to b23 Second balloon
Da Maximum outer diameter of the first balloon
Db1, Db2, Db11 to Db14, and Db21 to Db23 Maximum outer diameters of the second balloon h1 to hn Holes formed in the side wall of the inflation tube(s)
L10 Lumen of the shaft
L31 Lumen of the first inflation tube
L32, L32b1, L32b2 Lumens of the second inflation tubes
L40 Guidewire lumen
W1 Length of the first section in the longitudinal direction
W2 Length of the second section in the longitudinal direction
x Longitudinal direction
x1 Fixation position
y Radial direction
z Circumferential direction

## Claims

1. A balloon catheter comprising:
a shaft extending in a longitudinal direction from a proximal side to a distal side;
a first balloon and a second balloon each disposed at a distal part of the shaft; and
a first inflation tube and a second inflation tube each disposed in a lumen of the shaft,
wherein
the first inflation tube has a lumen in communication with the lumen of the shaft and a lumen of the first balloon,
the second inflation tube has a lumen in communication with the lumen of the shaft and a lumen of the second balloon,
the shaft has
a first section which is present on a distal side and in which the first inflation tube and the second inflation tube are disposed and
a second section which is present proximal to the first section and in which the first inflation tube and the second inflation tube are disposed,
at least two of the shaft, the first inflation tube, and the second inflation tube are fixed to each other in the first section, and
the shaft, the first inflation tube, and the second inflation tube are not fixed to each other in the second section.

2. The balloon catheter according to claim 1,
wherein a length W2 of the second section in the longitudinal direction is longer than a length W1 of the first section in the longitudinal direction.

3. The balloon catheter according to claim 2,
wherein a ratio (W2/W1) of the length W2 of the second section in the longitudinal direction to the length W1 of the first section in the longitudinal direction has a value of from 1.1 to 2.

4. The balloon catheter according to claim 1,
wherein the shaft further has, in the lumen thereof, a guidewire tube through which a guidewire is inserted, and
the guidewire tube is partially disposed in the lumen of the first balloon.

5. The balloon catheter according to claim 1,
wherein the first inflation tube has, in the second section, a side wall in which a hole is formed, the hole allowing the lumen of the first inflation tube and the lumen of the shaft to be in communication with each other.

6. The balloon catheter according to claim 1,
wherein the second balloon comprises a plurality of second balloons, and
the second balloons are arranged in a circumferential direction of an outer periphery of the first balloon.

7. The balloon catheter according to claim 6,
wherein the second inflation tube comprises a plurality of second inflation tubes, and
the first inflation tube has, in the second section, a side wall in which a hole is formed, the hole allowing the lumen of the first inflation tube and the lumen of the shaft to be in communication with each other, or
at least one of the plurality of second inflation tubes has, in the second section, a side wall in which a hole is formed, the hole allowing the lumen of the at least one second inflation tube and the lumen of the shaft to be in communication with each other.
